# EUROPEAN PATENT SPECIFICATION

(11) **EP 3 945 990 B1**
(45) Date of publication and mention of the grant of the patent: **26.11.2025**
(21) Application number: 20776941.5
(22) Date of filing: 25.03.2020
(51) Int. Cl.: A61B 1/00, A61B 1/04, A61B 1/045, H04B 5/00, A61B 5/07

(54) **A COMMUNICATION MODULE FOR AN IN-VIVO DEVICE**
KOMMUNIKATIONSMODUL FÜR EINE IN-VIVO-VORRICHTUNG
MODULE DE COMMUNICATION POUR DISPOSITIF IN VIVO

(30) Priority: 27.03.2019 US 201962824680 P
(43) Date of publication of application: 09.02.2022
(73) Proprietor: Given Imaging Ltd., 2069204 Yoqneam (IL)
(72) Inventor: DIUKMAN, Iddo, 3446706 Haifa (IL); GRUMAN, Baruch, 73133 Lapid (IL)
(74) Representative: Maschio & Soames IP Ltd
(86) International application number: PCT/IL2020/050358
(87) International publication number: WO 2020/194305

(56) References cited:
- WO-A2-2007/074447
- US-A1- 2007 156 016
- US-A1- 2011 285 835
- US-A1- 2012 262 560
- US-B2- 7 061 523
- US-B2- 8 786 691
- US-B2- 9 237 839

## Description

### TECHNOLOGICAL FIELD

The present invention is in the field of communication and transmission, in particular, referring to an ex-vivo communication module configured for communicating with a swallowable in-vivo device.

### BACKGROUND OF THE INVENTION

It is well known in the art to use swallowable in-vivo devices to monitor and detect pathologies in the GI tract. In its most common form, the in-vivo device comprises an in- vivo communication module configured for transmitting and receiving signals, for example, transmitting images of the GI tract captured by the in-vivo device and receiving orders relating to its operation.

The in-vivo device usually operates in conjunction with an ex-vivo device comprising an ex-vivo communication module, the in-vivo and ex-vivo communication modules forming together a communication system.

The ex-vivo device can be a portable device fitted to the patient to be in the closest possible proximity to the GI tract and the general area in which the in-vivo device is expected to be.

Acknowledgement of the above references herein is not to be inferred as meaning that these are in any way relevant to the patentability of the presently disclosed subject matter.

WO 2007074447 discloses an *in vivo* sensing system which includes an autonomous *in vivo* sensing device to sense a body lumen and to transmit sensed data; a communication device located externally to a patient's body to receive the transmitted data; and a recording device to record the received data, the recording device being connected to the communication device and comprising a controller to verify a version of the communication device that is connected to it. The autonomous *in vivo* sensing device may be an ingestible imaging capsule.

US 2012262560 discloses a device, system, and method for activating and initializing an *in vivo* imaging device with an RF radiation signal. Functionality of the *in vivo* imaging device is tested and results may be reported to a user. The *in vivo* imaging device may include an RF switch to facilitate powering or deactivation of one or more electrical components of the device. The initialization system may include an optical artifact testing unit, a field of illumination testing unit, and a transmission/reception testing unit. The activation system may comprise an *in vivo* device association unit which may relate a designated device to a single data recorder or to a single controller.

US 2007/156016 discloses a system and method for detecting and recording interference in an RF communication channel for an ingestible imaging device. During a period when transmission in a communication channel may be idle, interference levels may be detected and recorded. Changes in transmission levels may be altered based on the detected interference level. Transmission may be delayed due to detected levels of interfering signals.

### SUMMARY

The invention is defined in the appended claims.

In accordance with one example of the subject matter of the present application, there is provided an ex-vivo communication module configured for communicating with a swallowable in-vivo device, said communication module comprising a receiving unit configured for operating at a first frequency range for receiving signals from said in-vivo device, and a transmitting unit configured for operating at a second frequency range, different from said first frequency range (e.g. not including overlapping frequencies), for transmitting signals to said in-vivo device, wherein said transmitting unit also constitutes a secondary receiving unit, configured for receiving signals from the in-vivo device.

Hereinafter, the terms 'uplink unit' and 'downlink unit' may be used interchangeably with the terms 'receiving unit' and 'transmitting unit'. Specifically, the term 'downlink' refers to transmitting a signal to the in-vivo device while the term 'uplink' refers to receiving a signal from the in-vivo device.

The communication module is configured for being located external to the body, for example, placed on the body of the patient, for allowing proper communication between the module and the in-vivo device.

The receiving unit and the transmitting unit may be constituted by antennas, each antenna being designed for its own operation frequency range. In accordance with one example, the first frequency range may be at least one order of magnitude greater/smaller than the second frequency range. The transmitting unit may be configured for operating at 5-30MHz, more specifically at 10-20MHz, and even more specifically at 12-15MHz, while the receiving unit may be configured for operating at 350-550Mhz, more specifically at 400-500MHz, and even more specifically at 420-450MHz.

It should be appreciated that since the ex-vivo module and the in-vivo device need to communicate with each other, it would be desired to increase the transmission quality by using a low frequency range, both for the uplink and for the downlink, since low frequency range transmissions have a lower attenuation within the human body. However, since the in-vivo device is required to transmit a large amount of data (e.g. images obtained from the GI), using a low frequency as in the downlink antenna, may not provide sufficient bandwidth for such an amount of data. Therefore, while the downlink antenna may still be chosen to be at the above mentioned low frequency range of several MHz, the uplink antenna is chosen to be at a high frequency range of several hundred MHz.

In accordance with the above, it should be appreciated that the design of the downlink antenna is optimized for its specific frequency range, and would therefore not be expected to perform properly for receiving transmissions at a considerably different frequency range, e.g. as previously mentioned, one or more orders of magnitude higher/lower. Nonetheless, it is suggested, under the concept of the subject matter of the present application, to use the downlink antenna as a secondary uplink (receiving) antenna, contradictory to its originally intended design.

Owing to the unique arrangement of the above system, requiring close proximity between the ex-vivo device and the in-vivo device, using the downlink antenna as a secondary receiver was found surprisingly useful in providing at least some of the necessary uplink communication with the in-vivo device at the high frequency range.

In other words, while the frequency range of the downlink antenna is around 13MHz, and naturally, if used as a receiving unit, would not be useful as an antenna at the high frequency range (around 400MHz), it still functions as a receiving antenna at a close range, albeit with poorer results. Thus, the above communication module provides a configuration in which two antennas, designed for operation at two different frequency ranges (high frequency and low frequency) and for two different purposes, both cover the receiving end of an uplink communication with the in-vivo device (which transmits at a single predetermined high frequency range), thereby compensating for each other, posing a significant improvement in uplink communication.

In operation, the in-vivo device traveling along the GI tract of the patient constantly changes its distance from, and orientation with respect to, the ex-vivo communication module. While the transmissions of the in-vivo device are to be received by the uplink unit, the position and orientation of the in-vivo device may, at times, be such that the uplink unit does not properly receive the transmitted signal. It is at this point that the downlink unit comes into effect as a secondary uplink receiving unit, operating as a backup/complementary receiving unit. Thus, even if the uplink signal received by the downlink unit is of poor quality, it may still be better than receiving no signal at all by the uplink unit alone.

It is appreciated that at some instances, neither the downlink unit nor the uplink unit may receive any signal from the in-vivo device. However, during testing of the above uplink/downlink configuration, it was demonstrated that using the downlink unit as an uplink backup provides receiving a greater percentage of the in-vivo transmissions compared to the standard configuration, in which only the uplink unit is used for uplink reception.

The communication module comprises, according to the invention, a processor configured for providing input to the transmitting unit (which will then be transmitted to the in-vivo device), and for receiving data from the receiving unit. The communication module further comprises, according to the invention, a modem unit interposed between the processor and the receiving/transmitting unit, configured for providing communication therebetween.

The modem unit may be configured for applying a diversity scheme, whereby, according to the invention, it detects which of the units provides a better uplink transmission, and, upon such detection, chooses the better transmission of the two to be provided to the processor. In this manner, the quality of the data received from the in-vivo device may be optimized.

In accordance with one example, the uplink unit may have a dual connection with the modem, one for uplink and one for downlink, and the modem may be configured for continuously alternating between a downlink mode, providing data to the downlink unit and an uplink mode, receiving data from said uplink unit. Alternatively, in accordance with another example, both the uplink connection and the downlink connection of the uplink unit may be connected to the modem via a multiplexer, allowing continuous uplink/downlink communication between the modem and the uplink/downlink units.

The communication module may be incorporated within an ex-vivo device configured for being fitted to the patient. In accordance with one example, the ex-vivo device may be a patch configured for being applied to the patient's skin. In accordance with another example, the ex-vivo device may be a portable device configured for being carried by the patient, similar to a monitor. In accordance with still another example, the ex-vivo device may be a belt configured for being fitted to the patient and extend around his/her body, rather than having a pin-point location.

The uplink and downlink antennas may be flat, allowing at least part of the communication module to have a flat-sheet design, making it particularly suitable for incorporation into a patch or belt as suggested above. Specifically, a flat-sheet design of the communication module may allow it to bend and twist, thereby allowing it to assume the natural shape of the patient's body.

In accordance with one specific example, the communication module may have the shape of a rectangular flat-sheet design, having a length dimension L and a width dimension W. Under this design, the receiving antenna may be formed on the flat-sheet while the transmitting antenna may extend circumferentially along the edge of the flat-sheet. Specifically, the receiving antenna may be a monopole antenna.

In accordance with a specific design, the transmitting antenna may extend around the monopole uplink antenna, and be a coil-antenna, making several loops around the receiving antenna.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1A** is a schematic view of a patient's body fitted with a patch comprising the communication module of the present application;
**Fig. 1B** is a schematic view of the GI tract of the patient from Fig. 1A, containing an in-vivo device configured for communicating with the communication module of Fig. 1A;
**Fig. 2** is a schematic view of the communication module shown in Fig. 1A;
**Fig. 3** is a schematic view of the patch shown in Fig. 1A, comprising the communication module of the present application;
**Fig. 4A** is a schematic isometric view of a another example of a patch, comprising a communication module in accordance with the present application;
**Fig. 4B** is a schematic exploded view of the layers comprising the patch shown in Fig. 4A; and
**Fig. 4C** is a schematic front view of the communication module incorporated in the patch shown in Figs. 4A and 4B.

It will be appreciated that for simplicity and clarity of illustration, elements shown in the figures have not necessarily been drawn accurately or to scale. For example, the dimensions of some of the elements may be exaggerated relative to other elements for clarity, or several physical components may be included in one functional block or element. Further, where considered appropriate, reference numerals may be repeated among the figures to indicate corresponding or analogous elements.

### DETAILED DESCRIPTION OF EMBODIMENTS

In the following detailed description, numerous specific details are set forth in order to provide a thorough understanding of the invention. However, it will be understood by those skilled in the art that the present invention can be practiced without these specific details. In other instances, well-known methods, procedures, and components, modules, units and/or circuits have not been described in detail so as not to obscure the invention.

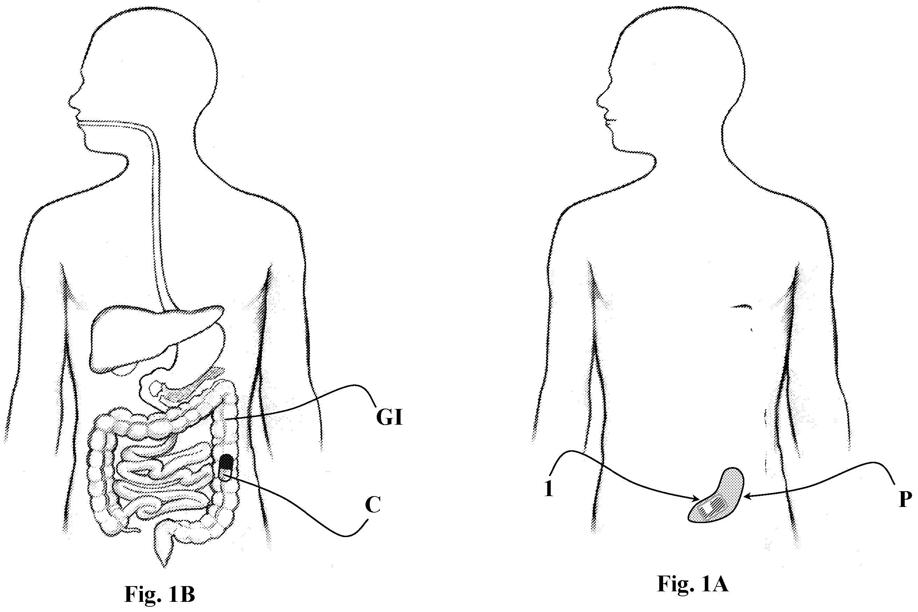

Attention is first drawn to Figs. 1A and 1B, in which a patient is shown having an in-vivo device in the form of a swallowable capsule C contained within their GI tract, and being fitted, externally, with a patch P comprising a communication module of the present application, generally designated 1.

The communication module 1 is configured for communicating with the swallowable capsule C during its travel along the GI tract, both receiving data from the capsule C (referred herein as 'uplink') and transmitting data to the capsule C (referred herein as 'downlink'). Specifically, the uplink data may contain images captured by the capsule C, parameters recorded thereby etc., while downlink data may include instructions sent to the capsule, for example, in order to change an operation mode of the capsule C, change its frame rate etc.

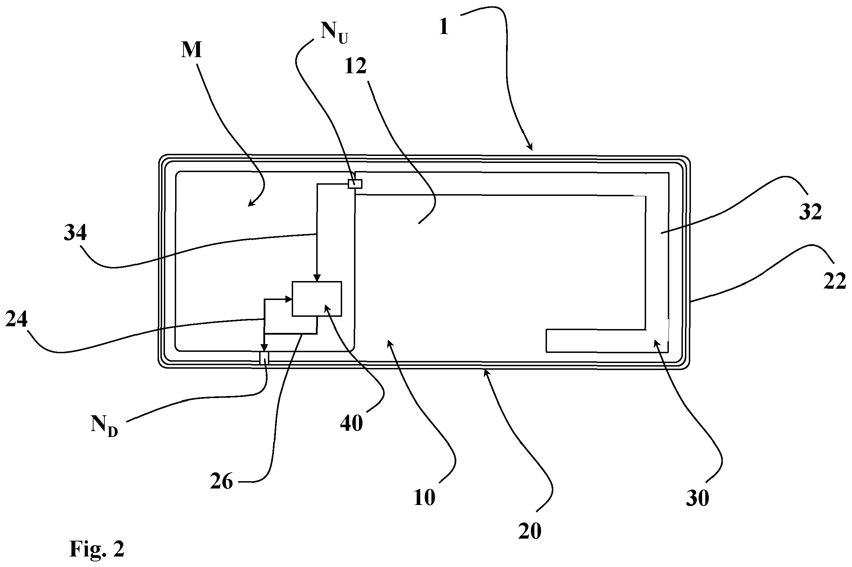

Turning now to Fig. 2, the communication module 1 is shown comprising a base 10 made of a flexible sheet of material 12 which is fitted with a board M, and having imprinted thereon a downlink antenna 20 extending circumferentially around the base 10 and an uplink antenna 30. The board M further comprises a modem unit 40 associated with both the uplink antenna 30 and the downlink antenna 20 via respective connections N_{U} and N_{D}.

The downlink antenna 20 is in the form of an antenna coil 22 and is configured for transmitting data to the capsule C at around 13.5MHz. The uplink antenna 30 is in the form of a monopole antenna 32 and is configured for receiving transmissions from the capsule C at around 435MHz. In addition, the downlink antenna 20 is also configured for receiving transmission from the capsule C, thereby operating as a secondary uplink antenna.

It should be noted that the capsule C is designed to transmit data at a high frequency range (hundreds of MHz), owing to bandwidth considerations, yielded by the requirement to transmit a large amount of data (e.g. in-vivo images). Therefore, the uplink antenna 30 is chosen to operate at a corresponding high frequency range, for optimizing reception from the capsule C. However, the downlink antenna 20 is not similarly limited in its transmission frequency, and can therefore be designed to operate at a low frequency range (tens of MHz), which reduces attenuation of the signal while passing through the tissue of the human body.

During travel of the capsule C within the GI tract, it may, at times, reach a location, assume a position or orientation which lead to the uplink antenna 30 being unable to properly receive the signal transmitted by the capsule C. In order to compensate for this, the downlink antenna 20 is used, despite being optimized for operating at a frequency range considerably different than that of the capsule's transmitter, and may even be able to better pick up the signal from the capsule C than the uplink antenna 30.

While the modem unit 40 is connected to the uplink antenna 30 only as a receiver via connection 34, it is connected to the downlink antenna 20 both as a transmitter via link 26 and as a receiver via link 24. Each of the uplink/downlink antennas 30, 20, may pick up a strong signal, a weak signal or no signal at all. The modem unit 40 is configured for operating under a diversity scheme, detecting which of the connections 24, 34 provides the stronger signal and prefer it to the weaker signal, leading to at least the following cases (the terms 'weak' and 'strong' used herein are used relatively to one another):

**Table 1.**

| | **Uplink antenna** | **Downlink antenna** | **Modem unit selects** |
|---|---|---|---|
| **1** | Strong signal | No signal | Signal from uplink antenna |
| **2** | Strong signal | Weak signal | Signal from uplink antenna |
| **3** | Weak signal | No signal | Signal from uplink antenna |
| **4** | Weak signal | Strong signal | Signal from downlink antenna |
| **5** | No signal | Strong signal | Signal from downlink antenna |
| **6** | No signal | Weak signal | Signal from downlink antenna |
| **7** | No signal | No signal | None |

It was clearly demonstrated, during testing of the above uplink/downlink configuration, that using the downlink unit as an uplink backup provides, statistically, receiving a greater percentage of the transmissions from the in-vivo capsule C compared to a configuration in which the downlink antenna is only used for downlink.

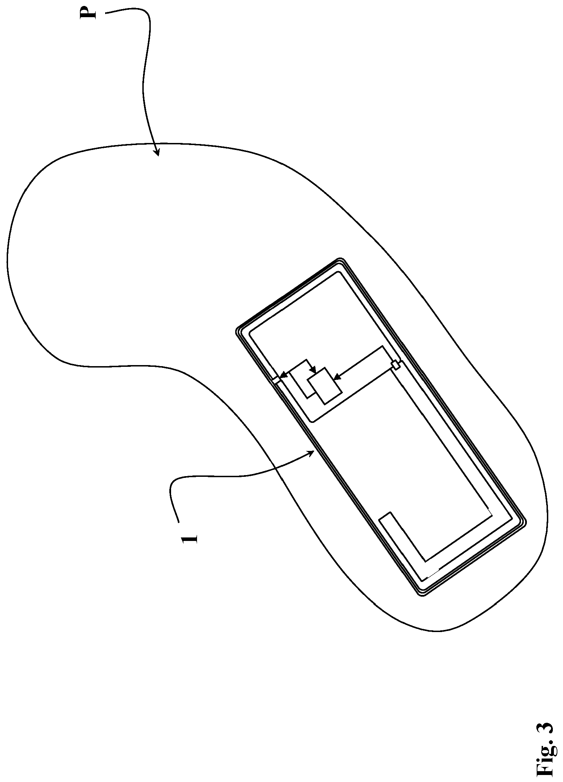

Turning now to Fig. 3, the communication module 1 may be incorporated within a patch P, which is, in turn, configured for being fitted to a patient's body by, for example, adhesion. It should be noted that the flexibility of the sheet 12 (and of the antennas 20, 30 printed thereon) may provide a significant advantage in terms of user comfort, since the patch is to be adhered to the body and thus provides a less limited movement on the side of the patient.

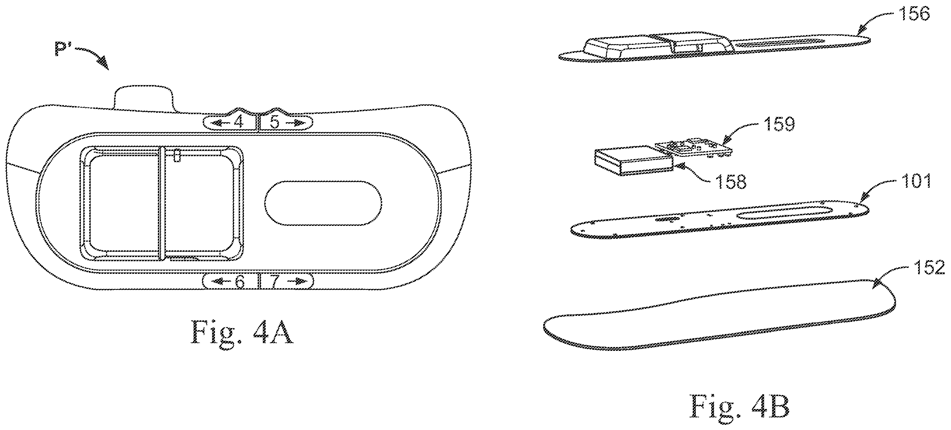

Turning now to Figs. 4A to 4C, another example of a patch according to some embodiments is shown, generally designated P', and including a plurality of functional layers including (but not limited to):
- an adhesive layer 152 configured for being in direct contact with the patient's body and for fixating the position of the patch with respect to the patient's body;
- a communication layer 101 constituting the communication module; and
- an external cover layer 156;

The patch 10 further comprises a power unit 158 and a processing unit 159 nested within the external cover layer 156.

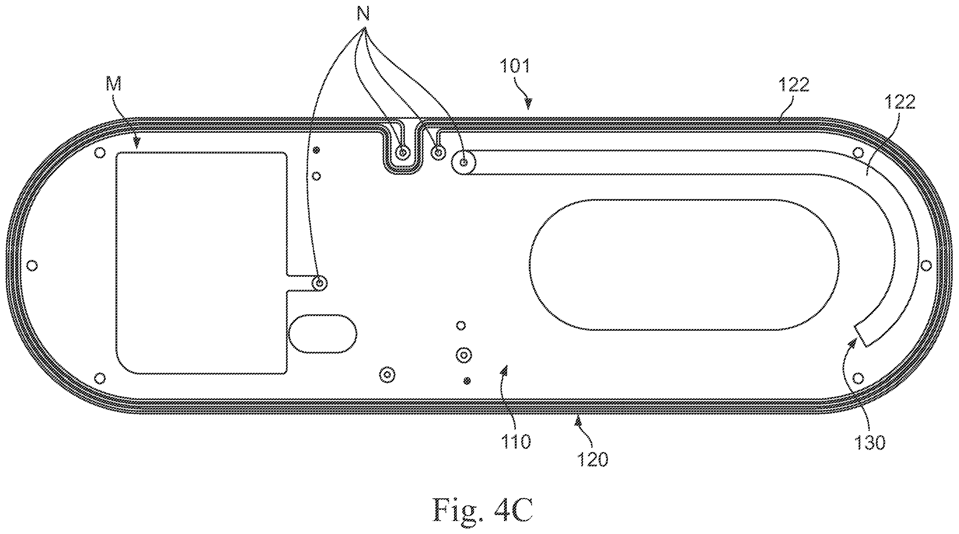

With particular reference being drawn to Fig. 4C, the scheme of the communication module 101 is shown comprising an oval base 110 made of a flexible sheet of material 112 which is fitted with a board M, and having imprinted thereon a downlink antenna 120 extending circumferentially around the oval base 110 and an uplink antenna 130. The board M and antennas 120, 130 are configured for being connected with one another via connection ends N.

The communication module 101 is essentially similar to the previously communication module 1, with the difference lying mostly in the oval design of the printed antenna (compared to the rectangular design of communication module 1), and in the design of the patch P'.

Those skilled in the art to which this invention pertains will readily appreciate that numerous changes, variations, and modifications can be made without departing from the scope of the invention, *mutatis mutandis.*

It will thus be seen that the objects set forth elsewhere herein, among those made apparent from the preceding description, are efficiently attained and, because certain changes may be made in carrying out the method described elsewhere herein and in the construction(s) set forth without departing from the spirit and scope of the invention, it is intended that all matter contained in the above description and shown in the accompanying drawings shall be interpreted as illustrative and not in a limiting sense.

In the foregoing detailed description, numerous specific details are set forth in order to provide an understanding of the invention. However, it will be understood by those skilled in the art that the invention can be practiced without these specific details. In other instances, well-known methods, procedures, and components, modules, units and/or circuits have not been described in detail so as not to obscure the invention. Some features or elements described with respect to one embodiment can be combined with features or elements described with respect to other embodiments.

Although embodiments of the invention are not limited in this regard, the terms "plurality" and "a plurality" as used herein can include, for example, "multiple" or "two or more". The terms "plurality" or "a plurality" can be used throughout the specification to describe two or more components, devices, elements, units, parameters, or the like. The term set when used herein can include one or more items. Unless explicitly stated, the method embodiments described herein are not constrained to a particular order or sequence. Additionally, some of the described method embodiments or elements thereof can occur or be performed simultaneously, at the same point in time, or concurrently.

## Claims

1. An ex-vivo communication module (1) configured for communicating with a swallowable in-vivo device (C), said communication module comprising a receiving unit (30) configured for operating at a first frequency range for receiving signals from said in-vivo device, and a transmitting unit (20) configured for operating at a second frequency range, different from said first frequency range, for transmitting signals to said in-vivo device, wherein said transmitting unit also constitutes a secondary receiving unit, configured for receiving signals from the in-vivo device;
wherein the communication module comprises a processor, wherein said processor is configured for at least the following: (a) providing input to the transmitting unit; and (b) receiving data from the receiving unit;
wherein said module further comprises a modem unit (40) interposed between the processor and the receiving/transmitting unit, configured for providing communication therebetween,
**characterised in that**
the modem unit is configured for detecting which of the receiving unit and the transmission unit provides a better transmission of signals received from said in-vivo device, and, upon such detection, to choose the better transmission of the receiving unit and the transmission unit to be provided to the processor.

2. A communication module according to Claim 1, wherein the receiving unit and the transmitting unit are constituted by antennas, each antenna being designed for operating at its own frequency range.

3. A communication module according to Claim 2, wherein the first frequency range is at least one order of magnitude greater/smaller than the second frequency range.

4. A communication module according to Claim 3, wherein the transmitting unit is configured for operating at 5-30MHz, more specifically at 10-20MHz, and even more specifically at 12-15MHz, and/or wherein the receiving unit is configured for data transfer operating at 350-550Mhz, more specifically at 400- 500MHz, and even more specifically at 420-450MHz.

5. A communication module according to Claim 1, wherein the transmitting unit may have a dual connection with the modem, one connection (34) for uplink and one connection (26) for downlink, wherein downlink refers to transmitting a signal from the communications module to the in vivo device and uplink refers to receiving a signal from the in vivo device at the communications module.

6. A communication module according to Claim 5, wherein the modem is configured for continuously alternating between a downlink mode, providing data to the transmitting unit and an uplink mode, receiving data from said receiving unit, or wherein both uplink connection and the downlink connection are connected to the modem via a multiplexer, allowing continuous uplink/downlink communication between the modem and the receiving/transmitting units.

7. A communication module according to Claim 5 or 6, wherein a receiving antenna (30) for the uplink connection and a transmitting antenna (20) for the downlink connection are both flat and are incorporated in a flexible sheet (12) of the communication module.

8. A communication module according to Claim 7, wherein the receiving antenna is a monopole antenna (32) formed on the sheet and/or wherein the transmitting antenna extends circumferentially along the edge of the sheet.

9. A communication module according to Claim 8, wherein the transmitting antenna extends around the monopole antenna.

10. A communication module according to Claim 9 wherein the transmitting antenna is a coil-antenna (22) making several loops around the monopole antenna.

11. A communication module according to any of Claims 7 to 10, wherein the flexible sheet allows the communication module to bend and twist, thereby allowing it to assume the natural shape of the patient' s body.

12. A communication module according to any one of Claims 1 to 11, wherein the communication module is incorporated within an ex-vivo device configured for being fitted to the patient.

13. A communication module according to Claim 12, wherein the ex-vivo device is: a) a patch (P) configured for being applied to the patient's skin; b) a portable device configured for being carried by the patient; or c) a belt configured for being fitted to the patient and extending around his/her body.

14. A communication module according to any one of Claims 1 to 13, wherein the communication module has the shape of a rectangular flat-sheet design, having a length dimension L and a width dimension W.

## Patentansprüche

1. Ex-vivo-Kommunikationsmodul (1), das für die Kommunikation mit einer verschluckbaren in-vivo-Vorrichtung (C) ausgelegt ist, wobei das Kommunikationsmodul eine Empfangseinheit (30), die für den Betrieb in einem ersten Frequenzbereich ausgelegt ist, um Signale von der in-vivo-Vorrichtung zu empfangen, und eine Übertragungseinheit (20), die für den Betrieb in einem zweiten Frequenzbereich ausgelegt ist, der sich von dem ersten Frequenzbereich unterscheidet, um Signale an die in-vivo-Vorrichtung zu übertragen, umfasst, wobei die Übertragungseinheit auch eine sekundäre Empfangseinheit bildet, die für den Empfang von Signalen von der in-vivo-Vorrichtung ausgelegt ist;
wobei das Kommunikationsmodul einen Prozessor umfasst, wobei der Prozessor für mindestens Folgendes ausgelegt ist: (a) Bereitstellen von Eingaben für die Übertragungseinheit; und (b) Empfangen von Daten von der Empfangseinheit;
wobei das Modul ferner eine Modemeinheit (40) umfasst, die zwischen dem Prozessor und der Empfangs-/Übertragungseinheit angeordnet ist und dafür ausgelegt ist, Kommunikation zwischen diesen bereitzustellen, **dadurch gekennzeichnet, dass**
die Modemeinheit dafür ausgelegt ist zu erkennen, welche der Empfangseinheit und der Übertragungseinheit eine bessere Übertragung der von der In-vivo-Vorrichtung empfangenen Signale bietet, und nach einer solchen Erkennung die bessere Übertragung von der Empfangseinheit und der Übertragungseinheit auszuwählen, die an den Prozessor bereitgestellt werden soll.

2. Kommunikationsmodul nach Anspruch 1, wobei die Empfangseinheit und die Übertragungseinheit aus Antennen bestehen, wobei jede Antenne für den Betrieb in ihrem eigenen Frequenzbereich ausgelegt ist.

3. Kommunikationsmodul nach Anspruch 2, wobei der erste Frequenzbereich mindestens eine Größenordnung größer/kleiner ist als der zweite Frequenzbereich.

4. Kommunikationsmodul nach Anspruch 3, wobei die Übertragungseinheit für den Betrieb bei 5-30 MHz, konkreter bei 10-20 MHz und noch konkreter bei 12-15 MHz ausgelegt ist und/oder wobei die Empfangseinheit für die Datenübertragung bei 350-550 MHz, konkreter bei 400-500 MHz und noch konkreter gesagt bei 420-450 MHz ausgelegt ist.

5. Kommunikationsmodul nach Anspruch 1, wobei die Übertragungseinheit eine doppelte Verbindung mit dem Modem aufweisen kann, eine Verbindung (34) für die Aufwärtsstrecke und eine Verbindung (26) für die Abwärtsstrecke, wobei sich Abwärtsstrecke auf das Übertragen eines Signals vom Kommunikationsmodul an die In-vivo-Vorrichtung bezieht und sich Aufwärtsstrecke auf das Empfangen eines Signals von der In-vivo-Vorrichtung am Kommunikationsmodul bezieht.

6. Kommunikationsmodul nach Anspruch 5, wobei das Modem dafür ausgelegt ist, kontinuierlich zwischen einem Abwärtsstreckenmodus, der Daten an die Übertragungseinheit bereitstellt, und einem Aufwärtsstreckenmodus, der Daten von der Empfangseinheit empfängt, zu wechseln, oder wobei sowohl die Aufwärtsstreckenverbindung als auch die Abwärtsstreckenverbindung mit dem Modem über einen Multiplexer verbunden sind, wodurch eine kontinuierliche Aufwärtsstrecken-/ Abwärtsstreckenkommunikation zwischen dem Modem und der Empfangs-/Übertragungseinheit ermöglicht wird.

7. Kommunikationsmodul nach Anspruch 5 oder 6, wobei eine Empfangsantenne (30) für die Aufwärtsstreckenverbindung und eine Übertragungsantenne (20) für die Abwärtsstreckenverbindung beide flach und in eine flexible Folie (12) des Kommunikationsmoduls integriert sind.

8. Kommunikationsmodul nach Anspruch 7, wobei die Empfangsantenne eine auf der Folie ausgebildete Monopolantenne (32) ist und/oder wobei sich die Übertragungsantenne in Umfangsrichtung entlang der Kante der Folie erstreckt.

9. Kommunikationsmodul nach Anspruch 8, wobei sich die Übertragungsantenne um die Monopolantenne herum erstreckt.

10. Kommunikationsmodul nach Anspruch 9, wobei die Übertragungsantenne eine Spulenantenne (22) ist, die mehrere Schleifen um die Monopolantenne bildet.

11. Kommunikationsmodul nach einem der Ansprüche 7 bis 10, wobei die flexible Folie ermöglicht, dass das Kommunikationsmodul gebogen und verdreht werden kann, wodurch es die natürliche Form des Körpers des Patienten annehmen kann.

12. Kommunikationsmodul nach einem der Ansprüche 1 bis 11, wobei das Kommunikationsmodul in eine Ex-vivo-Vorrichtung integriert ist, die zum Anbringen am Patienten ausgelegt ist.

13. Kommunikationsmodul nach Anspruch 12, wobei die Ex-vivo-Vorrichtung: a) ein Pflaster (P) ist, das zum Aufbringen auf die Haut des Patienten ausgelegt ist; b) eine tragbare Vorrichtung ist, die zum Tragen durch den Patienten ausgelegt ist; oder c) ein Gürtel ist, der zum Anlegen am Patienten und zum Umschlingen seines Körpers ausgelegt ist.

14. Kommunikationsmodul nach einem der Ansprüche 1 bis 13, wobei das Kommunikationsmodul die Form einer rechteckigen flachen Folie mit einer Längsabmessung L und einer Breitenabmessung W aufweist.

## Revendications

1. Module de communication ex vivo (1) configuré pour communiquer avec un dispositif in vivo pouvant être avalé (C), ledit module de communication comprenant une unité de réception (30) configurée pour fonctionner dans une première plage de fréquences pour recevoir des signaux provenant dudit dispositif in vivo, et une unité d'émission (20) configurée pour fonctionner dans une deuxième plage de fréquences, différente de ladite première plage de fréquences, pour émettre des signaux vers ledit dispositif in vivo, où ladite unité d'émission constitue également une unité de réception secondaire, configurée pour recevoir des signaux provenant du dispositif in vivo ;
où le module de communication comprend un processeur, où ledit processeur est configuré pour au moins : (a) fournir une entrée à l'unité d'émission ; b) recevoir des données de l'unité de réception ;
où ledit module comprend en outre une unité de modem (40) interposée entre le processeur et l'unité de réception/émission, configurée pour assurer la communication entre eux,
**caractérisé en ce que**
l'unité de modem est configurée pour détecter laquelle de l'unité de réception et de l'unité d'émission fournit une meilleure transmission des signaux reçus dudit dispositif in vivo et, lors de cette détection, pour choisir la meilleure transmission de l'unité de réception et de l'unité d'émission à fournir au processeur.

2. Module de communication selon la revendication 1, dans lequel l'unité de réception et l'unité d'émission sont constituées par des antennes, chaque antenne étant conçue pour fonctionner dans sa propre plage de fréquences.

3. Module de communication selon la revendication 2, dans lequel la première plage de fréquences est au moins un ordre de grandeur supérieur/inférieur à la deuxième plage de fréquences.

4. Module de communication selon la revendication 3, dans lequel l'unité d'émission est configurée pour fonctionner à 5-30 MHz, plus spécifiquement à 10-20 MHz, et encore plus spécifiquement à 12-15 MHz, et/ou dans lequel l'unité de réception est configurée pour un transfert de données fonctionnant à 350-550 Mhz, plus spécifiquement à 400-500 MHz, et encore plus spécifiquement à 420-450 MHz.

5. Module de communication selon la revendication 1, dans lequel l'unité d'émission peut avoir une double connexion avec le modem, une connexion (34) pour la liaison montante et une connexion (26) pour la liaison descendante, où la liaison descendante se rapporte à la transmission d'un signal du module de communication au dispositif in vivo et la liaison montante se rapporte à la réception d'un signal du dispositif in vivo au module de communication.

6. Module de communication selon la revendication 5, dans lequel le modem est configuré pour alterner en continu entre un mode de liaison descendante, fournissant des données à l'unité d'émission et un mode de liaison montante, recevant des données de ladite unité de réception, ou dans lequel à la fois la connexion de liaison montante et la connexion de liaison descendante sont connectées au modem par l'intermédiaire d'un multiplexeur, permettant une communication continue de liaison montante/descendante entre le modem et les unités de réception/émission.

7. Module de communication selon la revendication 5 ou la revendication 6, dans lequel une antenne de réception (30) pour la connexion de liaison montante et une antenne d'émission (20) pour la connexion de liaison descendante sont toutes deux plates et sont incorporées dans une feuille flexible (12) du module de communication.

8. Module de communication selon la revendication 7, dans lequel l'antenne de réception est une antenne unipolaire (32) formée sur la feuille et/ou dans lequel l'antenne d'émission s'étend de manière circonférentielle le long du bord de la feuille.

9. Module de communication selon la revendication 8, dans lequel l'antenne d'émission s'étend autour de l'antenne unipolaire.

10. Module de communication selon la revendication 9, dans lequel l'antenne d'émission est une antenne bobine (22) formant plusieurs boucles autour de l'antenne unipolaire.

11. Module de communication selon l'une quelconque des revendications 7 à 10, dans lequel la feuille flexible permet au module de communication de se plier et de se tordre, lui permettant ainsi de prendre la forme naturelle du corps du patient.

12. Module de communication selon l'une quelconque des revendications 1 à 11, dans lequel le module de communication est incorporé dans un dispositif ex vivo configuré pour être adapté au patient.

13. Module de communication selon la revendication 12, dans lequel le dispositif ex vivo est : a) un patch (P) configuré pour être appliqué sur la peau du patient ; b) un dispositif portable configuré pour être porté par le patient ; ou c) une ceinture configurée pour être adaptée au patient et s'étendre autour de son corps.

14. Module de communication selon l'une quelconque des revendications 1 à 13, dans lequel le module de communication a la forme d'une conception rectangulaire en feuille plate, ayant une dimension de longueur L et une dimension de largeur W.
